# EUROPÄISCHE PATENTSCHRIFT

(11) **EP 1 399 482 B1**
(45) Veröffentlichungstag und Bekanntmachung des Hinweises auf die Patenterteilung: **18.03.2009**
(21) Anmeldenummer: 02758218.8
(22) Anmeldetag: 06.06.2002
(51) Int. Cl.: C07K 16/18, G01N 33/574, G01N 33/577, G01N 33/68, A61K 39/395, A61K 47/48, A61P 35/00

(54) **BESTIMMUNG VON BONE-SIALOPROTEIN IN KÖRPERFLÜSSIGKEITEN FÜR ONKOLOGISCHE FRAGESTELLUNGEN**
DETERMINATION OF BONE-SIALOPROTEIN IN BODILY FLUIDS FOR ONCOLOGICAL PROBLEMS
DOSAGE DE LA SIALOPROTEINE OSSEUSE DANS DES LIQUIDES BIOLOGIQUES EN MATIERE D'ONCOLOGIE

(30) Priorität: 13.06.2001 EP 01114388
(43) Veröffentlichungstag der Anmeldung: 24.03.2004
(73) Patentinhaber: Immundiagnostik AG, 64625 Bensheim (DE)
(72) Erfinder: ARMBRUSTER, Franz Paul, Immundiagnostik AG, 64625 Bensheim (DE); KARMATSCHEK, Markus, 64625 Bensheim (DE); PAULSSON, Mats, Institut für Biochemie, 50931 Köln (DE)
(74) Vertreter: Benedum, Ulrich Max
(86) Internationale Anmeldenummer: PCT/EP2002/006219
(87) Internationale Veröffentlichungsnummer: WO 2002/100901

(56) Entgegenhaltungen:
- WO-A-00/62065
- WO-A-99/50666
- KARMATSCHEK M ET AL: "IMPROVED PURIFICATION OF HUMAN BONE SIALOPROTEIN AND DEVELOPMENT OF A HOMOLOGOUS RADIOIMMUNOASSAY" CLINICAL CHEMISTRY, AMERICAN ASSOCIATION FOR CLINICAL CHEMISTRY. WINSTON, US, Bd. 43, Nr. 11, 1997, Seiten 2076-2082, XP001063137 ISSN: 0009-9147
- FISHER L W ET AL: "ANTISERA AND CDNA PROBES TO HUMAN AND CERTAIN ANIMAL MODEL BONE MATRIX NONCOLLAGENOUS PROTEINS" ACTA ORTHOPAEDICA SCANDINAVICA, MUNKSGAARD, COPENHAGEN, DK, Bd. 66, Nr. SUPPL 266, 1995, Seiten 61-65, XP000937776 ISSN: 0001-6470
- DIEL I J ET AL: "Serum bone sialoprotein in patients with primary breast cancer is a prognostic marker for subsequent bone metastasis." CLINICAL CANCER RESEARCH: AN OFFICIAL JOURNAL OF THE AMERICAN ASSOCIATION FOR CANCER RESEARCH. UNITED STATES DEC 1999, Bd. 5, Nr. 12, Dezember 1999 (1999-12), Seiten 3914-3919, XP002230791 ISSN: 1078-0432
- WALTREGNY D ET AL: "INCREASED EXPRESSION OF BONE SIALOPROTEIN IN BONE METASTATES COMPARED WITH VISCERAL METASTATES IN HUMAN BREAST AND PROSTATE CANCERS" JOURNAL OF BONE AND MINERAL RESEARCH, NEW YORK, NY, US, Bd. 15, Nr. 5, Mai 2000 (2000-05), Seiten 834-843, XP009004698 ISSN: 0884-0431
- WITHOLD W ET AL: "BONE SIALOPROTEIN IN SERUM OF PATIENTS WITH MALIGNANT BONE DISEASES" CLINICAL CHEMISTRY, AMERICAN ASSOCIATION FOR CLINICAL CHEMISTRY. WINSTON, US, Bd. 43, Nr. 1, 1997, Seiten 85-91, XP002923940 ISSN: 0009-9147
- ROSENBROCK ET AL: "Changes of biochemical bone markers during the menopausal transition" Bd. 40, Nr. 2, Februar 2002 (2002-02), Seiten 143-151,
- FASSBENDER ET AL: "Serum levels of immunoreactive bone sialoprotein in osteoporosis: Positive relations to established biochemical parameters of bone turnover" IN VIVO, Bd. 14, 2000, Seiten 619-624,
- SHAARAWY & HASAN: "Serum bone sialoprotein: a marker of bone resorption in postmenopausal osteoporosis" SCAND J CLIN LAB INVEST, Bd. 61, 2001, Seiten 513-522,
- STÖRK ET AL: "Bone sialoprotein is a specific biochemical marker of bone metabolism in postmenopausal women: A randomized 1-year study" OSTEOPOROSIS, Bd. 11, Nr. 11, 2000, Seiten 790-796,
- LI ETAL: "Biological variability of serum immunoreactive bone sialoprotein" CLIN. LAB., Bd. 44, 1998, Seiten 553-555,

## Beschreibung

Die Erfindung betrifft die immunologische Bestimmung von 8one-Sialoprotein (BSP) in Körperflüssigkeiten zur Diagnose und Überwachung von Erkrankung des Knochenstoffwechsels und des Knochenaufbaus uns besondere zur Diagnose, Prognose, Prophylaxe und Therapie von Knochenmetastasen bei primären Karzinomen.

Das Bone-Sialoprotein (BSP) ist ein phosphoryliertes Knochen-Glykoprotein mit einer relativen Masse von ca. 80 kDa in der SDS-PAGE. Die DNA für BSP kodiert für eine Peptidsequenz von ca. 33 kDA (Fisher L.W. et al. (1990), J. Biol. Chem., 265,2347-51; US 5 340 934). BSP ist eines der wenigen Matrixproteine, deren Bildung auf das mineralisierende Gewebe wie Knochen, Dentin und kalzinierendem Knorpel beschränkt ist. Das BSP stellt ca. 1 bis 15 % der gesamten nicht-kollagenen Proteine in der Knochenmatrix. Es wird von jenen Zellen exprimiert, die an der Bildung von Dentin, Knochen und Knorpel beteiligt sind, z.B. von Osteoblasten, sich entwickelnden Osteozyten, hypertrophen Chondrozyten, Odontoblasten und Zementoblasten.

Daneben wird BSP auch von Trophoblasten in der Placenta sowie von einigen Typen von Krebszellen gebildet, z.B. bei Lungen-, Brust-, Prostata-, Schilddrüsen- und Neuroblastoma-Primär- und Sekundärtumoren, beim Multiplen Myelom und in Knochenmetastasen. Der Grad der Expression von BSP durch den Tumor korreliert eng mit der Schwere der Krebserkrankung (Waltregny D. et al., Increased expression of bone sialoprotein in bone metastases compared with visceral metastases in human breast and prostate cancers, in J. Bone Miner. Res., 2000, 15(5), 834-43; Bellahcéne, A. et al., *Bone sialoprotein expression in primary human breast cancer is associated with bone metastases development, in* J. Bone Miner. Res., 1996, 11, 665-670; Waltregny, D. et al., Prognostic value of bone sialoprotein expression in clinically localized human prostate cancer, in Journal of the National Cancer Institute, 1998, 90, 1000-1008; Bellahcéne, A. et al., Expression of bone sialoprotein in primary breast cancer is associated with poor survival, in Int. J. Cancer, 1996, 69, 350-353).

Für Dentin, Knochen und Knorpel werden BSP zwei Funktionen zugeschrieben. Es soll als Adhäsionsmolekül die Anheftung und Ausbreitung von Zeilen auf der Gewebematrix bewirken, Da es *in vitro* Kristallisationskeime für biologisches Apatit bildet, wird vermutet, dass es auch *in vivo* an den Mineralisationen beteiligt ist. Das Ausschalten des BSP-Gens in Knock-out-Mäusen führte zu keiner sichtbaren Störung der Skelettbildung und funktion.

in Tumoren wird BSP eine Beteiligung an der Mikrokalzifikation (Castronovo, V. et al., Evidence that breast cancer associated microcalcifications are mineralized malignant cells, in Int, J. Oncol., 1998, 12, 305-308) und der Besiedlung von Knochen durch metastasierende Tumorzellen zugeschrieben (Bellahcène, A, et al, Expression of bone sialoprotien in primary breast canceris associated with poor survival, in Int. J. Cancer, 1996, 69, 350-353). Die Höhle der Konzentration an BSP im Serum von Patienten mit primärem Karzinomen dient der Diagnostik, ob diese Patienten Knochenmetastasen besitzen oder solche wahrscheinlich vom primären Tumor ausgehen werden (Diplomarbeit von Frau Ina-Alexandra Meier, Entwicklung eines Radioimmunoassays zur Bestimmung von Bonesialoprotein (BSP), 1996, Darmstadt, Fachhochschule, FB Chemische Technologie; Dissertation von Herrn Markus Karmatschek, Isolierung von Bonesialoprotein aus humanem Knochen, Aufbau eines Radioimmunoassays zu dessen Messung im Serum, 1996; FB Biologie der Technischen Hochschule Darmstadt: Karmatschek M et al., improved purification of human bone sialoprotein and development of a homologous immunoassay, Clinical Chemistry 1997, 43:11, 2076-2082, Diel I.J. et al., Elevated bone sialoprotein in primary breast cancer patients is a potent marker for bone metastases; in Proceedings of ASCO, 1998, 17, Abstract 461; Diel I.J. et al., Serum bone sialoprotein in patients with primary breast cancer is a prognostic marker for subsequent bone metastasis, in Clin. Cancer Res., 1999, 5, 3914-19; DE 198 13 633; DE 198 21 533; WO 99/50666).

Nach neuen Hypothesen soll das BSP die Trophoblasten und BSP-produzierenden Tumorzellen vor einem Angritt des Immunsystem schützen. BSP bindet nämlich mit hoher Affinität den Faktor-H des Komplementsystems, der bekanntlich den alternativen Weg der Komplementlyse hemmt. Auch kann das BSP über die eigene Erkennungssequenz (Arginin-Glycin-Aspartat, RGD) spezifisch an die Integrinrezeptoren auf Zelloberflächen binden, Bei einer Expression von BSP sollen dann die Tumorzellen den Faktor-H Im Blut und in den Gewebsflüssigkeiten an ihre Zelloberfläche binden bzw. um sich herum konzentrieren. Ein derartiger BSP-Schutz vor dem Komplementsystem des Blutes der Mutter wird auch für die Trophoblasten in der Placenta vermutet (Fedarko N.S. et al., Factor H binding of bone sialoprotein and osteopontin enables tumor cell evasion of complement-mediated attack, in J. Biol. Chem., 2000, 275, 16666-16672; WO 00/62065)

Eine Funktion des BSP wird weiterhin bei der Angiogenese vermutet. Neben der Adhäsion von Osteoklasten und Osteoblasten an die Knochenmatrix - durch das Binden der RGD-Erkennungssequenz in der Matrix an die alpha(v)beta(3) integrin-Rezeptoren auf der Zeilwand - wird auch beobachtet, dass die Adhäsion, Ausbreitung und Orientierung der Endotheizeilen vermutlich von BSP vermittelt wird. Die Blutgefäßbildung um einen Tumor geht nämlich einher mit der BSP-Expression in den Tumorzeilen (Bellahcène A et al, Bone sialoprotein mediates human endothelial cell attachment and migration and promotes angiogenesis, in Circ. Res. 2000, 86(8), 885-91)

Das BSP steht somit im Mittelpunkt des Geschehens bei der Bildung von Tumoren und Metastasen. So kann die Bindung von BSP über die RGD-Sequenz an Vitronectin- bzw. lntegrin-Rezeptoren von Tumor- und Epitheizeilen durch Antagonisten gehemmt werden (US 6 069 158; US 6 008 213; US 5 849 865; van der Pluijm et al., Bone-sialoprotein peptides are potent inhibitors of breast cancer cell adhesion to bone in vitro, in Cancer Res., 1996, 56, 1948-1955). EP 1 084 719 A1 lehrt eine pharmazeutische Zusammensetzung mit BSP als Wirksubstanz zur Unterstützung der Reparatur geschädigten Knochen- und Bindegewebes. WO 94/13310 lehrt eine Zusammensetzung mit einem BSP-Bindeprotein aus Staphylococcus aureaus als Wirkstoff. WO 00/36919 offenbart regulatorische Elemente zur zielgerichteten Kontrolle bzw. Unterdrückung der Expression von BSP in Tumor- und Bindegewebszellen, welche die Kalzifizierung fördern.

Da freies BSP in Körperflüssigkeiten vom Komplementfaktor-H mit hoher Affinität gebunden wird und das BSP an verschiedene Rezeptoren binden kann, ist sein Nachweis problematisch: So wurden gegen verschiedene Peptidteilstrukturen des BSP (Fisher, L.W et al., Antisera and cDNA probes to human and certain animal model bone matrix noncollagenous proteins. Acta Orthop Scand Suppl. 1995, 266, 61-655), gegen rekombinantes BSP (Stubbs JT 3rd et al., et al., Characterization of native and recombinant bone sialoprotein: delineation of the mineral-binding and cell adhesion domains and structural analysis of the RGD domain.J. Bone Miner. Res. 1997, 12(8), 1210-22) und gegen aus Knochen isoliertes BSP Antikörper im Kaninchen hergestellt, die alle BSP in humanem Serum nicht erkennen können. Erst nach Auftrennung der Serumproteinen über SDS-PAGE kann mit diesen Antikörpern BSP auf Westemblots nachgewiesen werden. Das erheblich größere Faktor-H-Molekül (150 kDa im Vergleich zu ca. 65 Kda) maskiert BSP im Serum vermutlich so weit, dass Antikörper nicht binden können. Außerdem liegt Faktor H im Serum im Überschuss vor (0,5 mg Faktor H / mL im Vergleich zu BSP mit < 20 ng / ml Serum beim gesunden Menschen und max. 160 ng/ ml bei Tumorpatienten). Es wird beansprucht, dass der immunologische Direktnachweis von BSP in Körperflüssigkeiten wegen der Bindung an den Komplementfaktor-H ohne reduzierende Probenaufbereitung unmöglich ist (Fedarko N.S. et al., Factor H binding of bone siatoprotein and osteopontin enables tumor cell evasion of complement-mediated attack, in J. Biol. Chem., 2000, 275, 16666-16672; WO 00/062065). Nach unseren Untersuchungen erlaubt eine derartige Probenaufbereitung bzw. Proteinspaltung erlaubt zwar eine mengenmäßige Bestimmung des vorhandenen BSP in Körperflüssigkeiten, die so erlangten Werte lassen aber keine Antworten zu bei onkologischen Fragestellungen.

Es ist Aufgabe der Erfindung, Antikörper gegen BSP für Fragestellungen in Zusammenhang mit der Prognose und Diagnose einer Fernmetastäsierung von primären Karzinomen in Knochen, zur Diagnose von Knochenmetastasen und für medizinische Anwendungen zur Verfügung zu stellen.

Diese Aufgabe wird gelöst durch Antikörper nach Anspruch 1 und deren Verwendung zur Bestimmung von BSP in Körperffussigkeiten. Die Antikörper gegen das humane Bone-Sialoprotein (BSP) können insbesondere Epitope binden, die auf humanem Bone-Sialoprotein aus Tumorzellen vorhanden sind, dessen posttranslationale Giykasylierung im Bereich der Aminosäuren 120 bis 135 (SWISSPROT: SIAL_HUMAN, Acc.No. P21815 ), beinhaltend die Aminosäuren TGLAA, gegenüber normalem Bone-Sialoprotein aus Knochen verändert oder unvollständig ist. Sie können erzeugt werden mit einem chemisch oder natürlich in der Glykosylierung veränderten Bone-Sialoprotein als Antigen, bevorzugt mit einem Bone-Sialoprotein aus Tumorzellen. Das in der Glykosylierung veränderte Bone-Sialoprotein kann also gentechnisch in Tumorzellen hergestellt werden. Sie können auch erzeugt werden gegen ein peptidisches Antigen, umfassend die Aminosäuresequenz TGLAA oder YTGLAA, ggf. gekoppelt an ein Trägerprotein. In einer anderen Ausführungsform werden die Antikörper hergestellt durch ein in der Glykosylierung verändertes Bone-Sialoprotein aus Knochenmaterial, dessen Spender nicht zur normalen Glykosylierung von Knochenproteinen fähig war. Wegen ihrer Nichtbeteiligung an der Komplementreaktion sind IgY-Antikörper aus Huhn besonders bevorzugt, insbesondere wenn diese Antikörper human oder humanisiert sind.

Die erfindungsgemäßen Aritikörper können eingesetzt werden in einem Verfahren zur Bestimmung von Bone-Sialoproteln aus Tumorzellen in Körperflüssigkeiten, insbesondere in Serum. Auf diese Weise kann eine Diagnostik und Prognostik von Knochenmetastasen erfolgen. Die Antikörper können selbstverständfich auch zur Diagnostik und ggf. zur Herstellung eines Medikaments verwendet werden, entweder als Wirksubstanz - bspw. zur Prophylaxe und Therapie von Knochenmetastasen oder auch als Targeting-Mittel zur Herstellung eines Diagnostikmittefs oder Medikaments.

Gegenstand der Erfindung ist weiterhin die Lokalisationsdiagnostik von Tumoren und Metastasen über Immunszintigraphie durch die erfindungsgemäßen Antikörper: Dabei werden die Anti-BSP Antikörper radioaktiv markiert und in die Blutbahn des Patienten injiziert. Sie binden spezifisch an Tumor- und Metastasengewebe und ihre Verteilung im Körper kann dann z.B, über einen Szintiscanner bildlich dargestellt werden.

Für die Bindung an den Komplex aus Faktor H und BSP müssen die Antikörper Epitope von BSP erkennen, welche nicht durch den Bindungspartner maskiert sind. Die Herstellung von solchen Antikörper war bisher nicht möglich. Die Erfindung stellt solche Antikörper zur Verfügung, weil die Antikörper gegen eine Isoform des gefalteten Sone-Siafoprotein (BSP) gerichtet sind und an Epitope binden, die nur von einem gefalteten Bone-Siafoprotein aus Tumorzellen gebildet werden, dessen Glykosylierungen im Bereich der Aminosäuren. 120 bis 135, umfassend die Aminosäuresquenz TGLAA oder YTGLAA, gegenüber dem normalem Bone-Sialoprotein aus Knochen verändert oder unvollständig sind oder fehlen. Normalerweise lassen sich keine spezifischen Antikörper gegen posttranslationale oder komplexe Zuckerstrukturen auf Proteinen erhalten, denn derartige Zuckerstrukturen werden in gleicher Weise und Form auf vielen verschiedenen Proteinen angefügt Entsprechend reagieren Antikörper gegen bestimmte Zuckerstrukturen mit vielen verschiedene Proteinen und gelten dann in der Regel als unspezifisch und wertlos. Anders beim Bone-Sialoprotein aus Tumorzellen. Die veränderte bzw. fehlende Zuckerstruktur bewirkt eine andere Faltung des Bone-Sialoproteins und schafft neue Epitope, an denen sowohl Aminosäuren bzw. Peptidstruktur als auch die vielfältigen verbliebenen Zuckerreste beteiligt sind. Diese Epitope sind aber charakteristisch für BSP aus entarteten Tumorzellen.

Antikörper gegen diese Epitope können erzeugt werden mit einem chemisch oder natürlich in der Glykosylierung veränderten BSP als Antigen, und gegebenenfalls durch Aufreinigung bzw. Absorption an der Isoform des Knochen-BSP. Bevorzugt werden die Antikörper hergestellt unter Verwendung von BSP aus Tumorzellen als Antigen. Nachdem das BSP aus Tumorzellen schwer hinreichenden Mengen isoliert werden kann, ist die gentechnische expression von in der Glykosylierung verändertem BSP in Tumorzellen die Methode der Wahl. Es wurde auch gefunden, dass einige Patienten in der Glykosylierung verändertes BSP im Knochenmaterial enthalten. Das heißt, diese zumeist sehr alten und an schwerer Osteoporose leidenden Patienten produzierten ein BSP, das zumindest in Teilen nicht normal glykosyliert war. Auch dieses BSP ist prinzipiell als Antigen zur Gewinnung der erfindungsgemäßen Antikörper geeignet. Die Isolierung der partiell glykosylierten Isoform, welcher der Tumor-Isoform des BSP gleicht, kann analog beschriebener Verfahren erfolgen (Karmatschek M et al., Improved purfication of human bone sialoprotein and development of a homologous radioimmunoassay, in Clin Chem. 1997, 43(11), 2076-82).

Die Antikörper können hergestellt werden in Maus, Meerschweinchen, Kaninchen, Hund Ziege, Schwein; Mensch, Esel oder Pferd, aber auch in allen Säugetieren. Besonders bevorzugt ist die Immunisierung von Vögeln, insbesondere Huhn, da sich hier wegen der großen abstammungsgeschichtlichen Unterschiede besonders leicht Antikörper gegen die Tumor-Isoform des BSP erhalten lassen. Zudem führt die Gegenwart IgY -Antikörper nicht zu einer Aktivierung des Komplementsystems, was wegen der möglichen Bindung zwischen Faktor-H und BSP problematisch sein kann. Die erfindungsgemäßen Antikörper erkennen die Tumor-Isoform des BSP in der Bindung mit Faktor-H.

Gegenstand der Erfindung sind somit spezifische Antikörper gegen die von Tumoren gebildeten Isoformen und ihre Nutzung für eine Antikörpertherapie oder Immunszintigraphie. Als Nebenwirkungen, die durch Anti-BSP Antikörper hergerufen werden, kommen in Frage: Direkte und indirekte Schädigung der Knochen und des Zahnbeins durch Aktivierung des Immunsystems gegen die Knochenmatrix und Knochenzellen und/oder direkte Zerstörung, bei Verwendung von Konjugaten der Antikörper mit Zellgiften oder Radioisotopen. Weiterhin ist eine Immunszintigraphie mit Anti-BSP-Antikörpern undenkbar, welche an die Knochenmatrix binden. Die Matrix würde radioaktiv markiert und die Lokalisierung von Tumoren wäre unmöglich.

Die für Tumor-BSP spezifischen Antikörper eignen für eine Tumortherapie und - Lokalisierung, da sie nicht oder nur in geringem Ausmaß an die Knochenmatrix oder an BSPproduzierende Zellen des Skeletts und des Zahnbeins binden. In einer besonders bevorzugten Anwendung der Erfindung werden Antikörper eingesetzt, welche spezifisch für Tumor-BSP sind und zusätzlich BSP im Komplex mit Faktor H erkennen. Nach Applikation von solchen Antikörpern in Tumorpatienten wird im Blut und in Gewebsflüssigkeit vorhandenes freies und an Faktor H gebundenes Tumor-BSP neutralisiert und damit der Schutz gegen die Komplementaktivierung aufgehoben, Tumorzellen spezifisch für die Zerstörung durch das Immunsystem markiert (z.B. durch klassische Aktivierung der Komplementkaskade) und Nebenwirkungen wie z.B. durch Aktivierung des Immunsystems gegen die Knochenmatrix oder das Zahnbein vermieden. In einer weiteren Anwendung der Erfindung werden humane polyklonale Anti-BSP Antikörper aus dem Ei von transgenen Hühnern mit humansiertem Immunsystem isoliert

Ebenfalls geeignet sind monoklonale Antikörper aus der Maus oder dem Huhn, welche die oben beschriebenen Bedingungen erfüllen und durch ein Screening gewonnen werden können, In einer speziellen Anwendung des Patentes werden hierzu die in Beispiel beschriebene monoklonale Zelllinie verwendet Weiterhin geeignet sind durch Fragmente von Antikörpern wie z.B. proteolytisch oder gentechnisch gewonnene Fab Fragmente..

Besonders geeignet sind humanisierte poly- und monoklonale Antikörper, die BSP im Komplex mit Faktor H erkennen und nicht an BSP in der Knochenmatrix binden. Bei der Verwendung von Antikörpern der Maus und des Huhns ist zwar ein besonderer therapeutischer Effekt durch Bildung humaner Anti-Maus-Antikörper (HAMA) oder Anti-Huhn-Antikörper (HACA) zu erwarten. HAMAs und HACAs können eine Immunantwort des Organismus auf das Tumorantigen induzieren und verstärken. Bei der Bestimmung von Tumormarkern entstehen jedoch Interferenzen mit den HAMAs und HACAs, die die in vitro Messmethode stören. Auf diese Weise kommt es zu falsch hohen Meßwerten für Tumormarker. Dies tritt nach Immunszintigraphie oder Immuntherapie mit entsprechenden Antikörpern auf, so daß eine korrekte Tumormarkerbestimmung erst nach Absorption der HAMAs oder HACAs in vitro erfolgen kann.

Diese Effekte können durch Verwendung von humanisierten Antikörpern ausgeschaltet werden. Polyklonale humanisierte Anti-BSP Antikörper können z.B. durch Immunisierung von transgenen Hühnern mit BSP gewonnen werden, bei denen in den embryonalen Stammzellen der Genbereich für den hühnerspezifischen Fc Teil des Immunglobulin (IgY) durch einen humanspezifischen ausgetauscht wurde (US 5 340 740; US 5 656 479). Die humanisierten Antikörper werden dann in den Eiern der Hühner abgelegt und können aus dem Eidotter isoliert werden (Mohammed S.M. et al., Deposition of genetically engineered human antibodies into the egg yolk of hens. Immunotechnology, 1998, 4: 115-125)

Zur Herstellung von humanisierten monoklonalen Antikörpern können Hybridomäzellen der Maus oder des Huhns mit geeigneten Anti-BSP Antikörpern nach Standardmethoden gewonnen werden und aus dem in diesen Zellen enthaltenem Genmaterial durch Rekombination humanisierte Antikörper entwickelt werden (US 5 585 089; US 5 565 332; US 5 225 539; US 5 693 761; US 5 585 089; US 5 530 101).

Es werden nun weitere Merkmale und Vorteile der Erfindung mit Bezug auf die Beispiele und die anliegenden Abbildungen beschrieben. Es zeigt
- Fig. 1: Westemblot von tumor- und knochenspezifischen Isoformen des BSP;
- Fig. 2: Westemblot des Zellkulturüberstands von untransfizierten EBNA-293-zellen (Negativkontrolle) und transfizierter EBNA-293-Zellen mit den Expressionskonstrukten GST-EK-BSP und his₆-myc-EK-BSP unter Verwendung eines monoklonalen Maus-Anti-BSP Antikörpers;

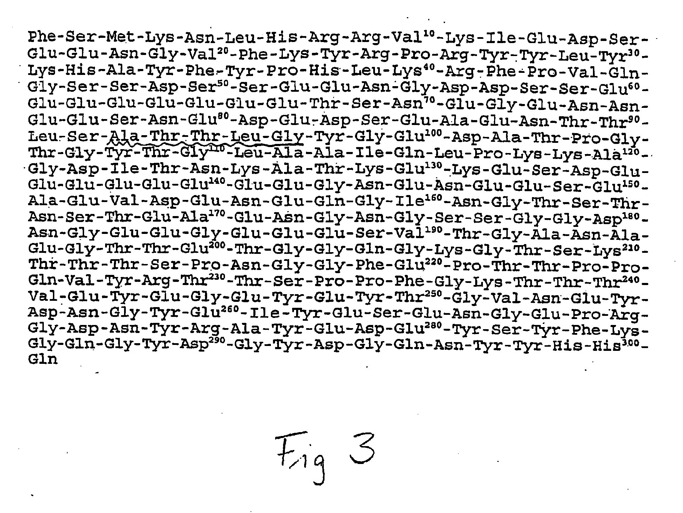

- Fig. 3: die Aminosäuresequenz von sekretiertem BSP nach Fisher et al (1991).
- Fig. 4: Muster eines Computerausdrucks für eine Mess-Eichkurve.

### Beispiel 1 - Charakterisierung von tumorspezifischen und knochenspezifischen BSP Isoformen im Westemblot

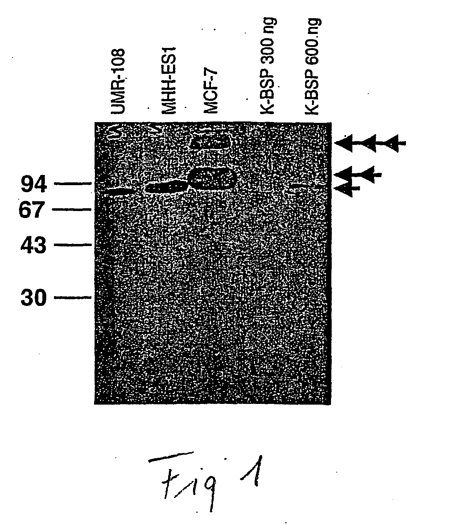

Serumfreie Überstände der humanen Osteosarkomzellinien UMR-108, MHH-ES1 und der Brustkrebszellinie MCF-7 (Ostrogenrezeptor positiv) als auch aus Knochen gereinigtes humanes BSP (K-BSP) wurden mittels SDS-PAGE auf einem 10 % Gel unter reduzierenden und denaturierenden Bedingungen aufgetrennt und elektrophoretisch auf Nitrozellulose übertragen. Die Membran wurde mit dem monoklonalen Maus Antikörper inkubiert Die Detektion des BSP erfolgte über einen an Peroxidase gekoppelten Anti-Maus-Antikörper der Ziege und Chemoluminiszenz-Detektion auf einem Röntgenfilm. Das Ergebnis ist in Abb. 1 dargestellt. Molekulargewichte und Laufstrecken der Marker sind auf der linken Seite angegeben. Die einzelne und doppelte Pfeilspitze zeigen das unterschiedliche Laufverhalten von Knochen/Osteosarkom-BSP und MCF-7-BSP. Letzteres enthält zusätzlich eine hochmolekulare Bande (Tripelpfeil), die in den anderen Spuren abwesend ist. BSP aus einer Tumorzelllinie weist somit ein deutlich höheres Molekulargewicht als BSP aus Knochen und aus Osteosarkomzellinien auf, wobei außerdem eine zweite Isoform mit noch höherem Molekulargewicht zu beobachten ist.

### Beispiel 2 - Herstellung von polyklonalen Antikörpern durch Immunisierung von Hühnern mit Knochen-BSP und BSP-Peptidteilstrukturen

Hühner und Kaninchen wurden mit BSP immunisiert, das nach dem von Karmatschek et al. beschriebenen Verfahren von Patienten isoliert worden war.

Aus den Eidottern und den Seren wurden polykonale Immunglobuline isoliert und in einem ElISA Verfahren gegen verschiedene Peptidteilstrukturen des BSP auf Bindung getestet. Tabelle 1 zeigt die Ergebnisse dieses Epitopmapping. Dabei wurden Peptidteilstrukturen aus der insgesamt 317 Aminosäuren langen Peptidsequenz des preproBSP (inkl. Leadersequenzen) chemisch synthetisiert, an eine Mikrotiterplatte gebunden und die Antikörper auf der Platte inkubiert. Der Test auf Bindung erfolgte nach Inkubation mit einem Konjugat von Peroxidase mit Anti-IgY- bzw Anti-Kaninchen-IgG-Immunglobulinen und nachfolgender Enzymreaktion durch Umsetzung eines Chromogens als Substrat.

**Tabelle 1**

| *Epitopmapping der gewonnenen anti-BSP-IgG und -IgY* | | | |
|---|---|---|---|
| Lage der Peptidteilstruktur im BSP | Aminosäuresequenz | Reaktionsstärke ELISA | |
| | | IgY | Kaninchen IgG |
| 112-123 | LeuGlyTyrGlyGluAspAlaThrProGlyThrGly | - | ? |
| 216-227 | GluThrGlyGlyGlnGlyLysGIyThrSerLysThr | - | ? |
| 300-311 | PheLysGlyGlnGlyTyrAspGlyTyrAspGlyGln | - | ? |
| 130-144 | IleGlnLeuProLysLysAlaGlyAsplleThrAsnLysAlaThr | +/- | + |
| 124-138 | TyrThrGlyLeuAlaAlalleGlnLeuProLysLysAlaGlyAsp | - | ++ |
| 137-151 | GlyAspIleThrAsnLysAlaThrLysGluLysGluLysGlu- SerAspGlu | - | + |
| 280-317 | SerGluAsnGlyGluProArgGlyAspAsnTyrArgAlaTyr- GluAspGluTyrSerTyrPheLysGlyGlnGlyTyrAspGly- TyrAspGlyGlnAsnTyrTyrHisHisGln | ++ | + |
| Humanes Knochen-BSP | | +++ | +++ |

Die Ergebnisse zeigen, dass die gewonnenen Hühnerantikörper bevorzugt an die C-terminale Sequenz des BSP binden, während die Kaninchenantikörper über einen größeren Bereich binden.

Weiterhin wurden polyklonale Antikörper (A0001) durch Immunisierung von Kaninchen mit der Peptidteilstruktur TyrThrGlyLeuAlaAlalleGlnLeuProLysLysAlaGlyAsp (Position 124-138) des BSP gewonnen, die bevorzugt an diese Peptidteilstruktur, aber auch spezifisch mit humanem Knochen-BSP reagieren.

Polyklonale Antikörper (AK_tBSP) jedoch, die durch Immunisierung von Kaninchen mit den Peptidteilstrukturen ThrGlyLeuAlaAla (Position 125-130) bspw TyrThrGlyLeuAlaAla (Position 124 bis 130) gewonnen wurden, das heißt nach Kopplung an Rinder-Thyreogiobulin als Träger, reagierten zwar mit der synthetischen Peptidteilstruktur, nicht aber mit human Knochen-BSP. Diese Antikörper erkennen überraschenderweise ausschließlich BSP aus Tumorzellen.

Für die Versuche wurden weiterhin die polyklonalen Antikörper A002 (erhalten von L.W.Fisher) und A003 (erhalten von Dr. van Ryden) verwendet. Diese Antikörper wurden nach Immunisierung mit den Peptidteilstrukturen TyrGluSerGluAsnGlyGluProArgGlyAsp-AsnTyrArgAlaTyrGluAsp (A002) bzw. LeuLysArgPheProValGlnGlyGly gewonnen. Ersteres Peptid stammt vom C-Terminus des BSP (Position 278-295) des BSP und enthält die RGD (ArgGlyAsp)-Erkennungssequenz des BSP für Rezeptoren des Integrin-Typs. Letzteres Peptid stammt vom N-Terminus der BSP-Primärstruktur. Auch diese Peptide erkannten bevorzugt die jeweiligen Teilstrukturen und reagierten spezifisch mit humanem Knochen-BSP.

### Beispiel 3 - Herstellung von rekombinantem BSP aus Brustkrebszellen für die Immunisierung

Aus dem Plasmid B6-5g (Fisher LW. et al., Human bone sialoprotein. Deduced protein sequence and chromosomal localisation, in J. Biol. Chem., 1990, 265(4), 2347-51) wurde die komplette cDNA für humanes BSP (ohne Signalpeptid) mittels PCR amplifiziert und in den episomalen eukaryotischen Expressionsvektor pCEP-Pu (Kohfeldt E et al., Properties of the extracellular calcium binding module of the proteoglycan testican; in FEBS Lett. 1997, 414(3), 557-61) kloniert. Die Primer waren wie folgt:
Nhe I BSP (sense): 5'-GCCCGCTAGCCTTCTCAATGAAAAATTTGCATCG-3'
Not I BSP (antisense): 5'-CAATGACTGCGGCCGCTCACTGGTGGTGGTAGTAATTC-3"

Die mit den Primern eingefügte Nhe I- und Not I-Schnittstellen wurden für die Klonierung in den Expressionsvektor. PGEP-PU benötigt. Dieser Vektor ist zudem zur Erleichterung der Proteinaufreinigung am 5'-Ende der multiplen Klonierungsstelle mit verschiedenen tags (z.B. His, Myc, G8T) ausgestattet, Diese tags können nach Aufreinigung des Proteins mit einer Protease (z.B. Faktor X oder Enterokinase) abgespalten werden. Die Einhaltung des korrekten Leserahmens wurde mittels Sequenzierung überprüft.

Die Expressionskonstrukte wurden mittels Liposomen-vermittelter stabiler Transfektion (FUGENE-Transfektlonsreagenz der Firma Roche) unter anderem in folgende humane Zellinien eingeführt:
- die embryonale Nierenzellinie EBNA-293
- die Osteosarkomzeilinien SAOS-2 und MG-63
- die humane Brustkrebszellinie MCF-7.

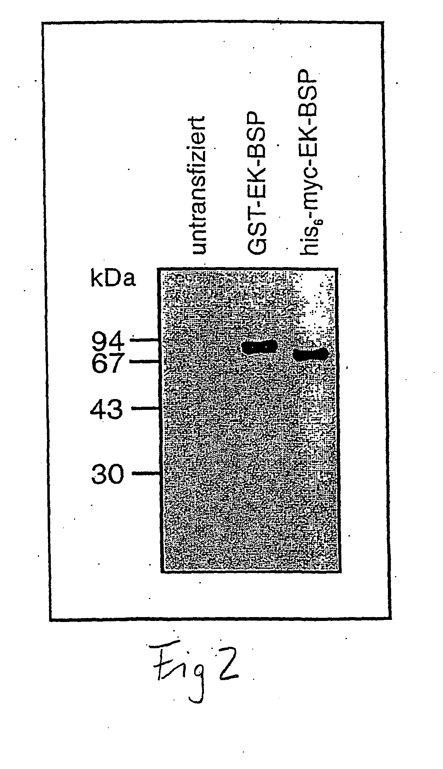

Eine rekombinanten Expression wurde nur in MCF-7 und EBNA-293-Zellen erhalten (siehe Figur 2). Die Osteosarkomzeillinen exprimiert auch nach wiederholten Transfektionsversuchen nicht.

### Beispiel4 - Analyse der Glykosylierung von rekombinantem BSP aus entarteten Zellen und Knochen-BSP

Transiente Zellen wurde 48 Stunden nach Transfektion zwei Tage in serumfreiem Medium kultiviert. Damit die Proteine Im FCS die Aufreinigung des rekombinanten BSP nicht erschwerten, wurden BSP-exprimierende Zellen nach Erreichen der Konfluenz unter serumfreien Bedingungen kultiviert. Unter diesen Bedingungen konnten nur EBNA-293 Zellen länger als 2 bis 4 überieben. Die Expression des rekombinanten BSP wurde durch SDS-PAGE und Immunoblots kontrolliert.

Die Untersuchung von serumfreien Zellkulturüberständen ergab mit all diesen Zelilinien im Westernblot positive Signale sowohl in Bezug auf BSP wie auch auf die Anwesenheit der verschiedenen tags.

2,5 Liter serumfreier Kulturüberstand der transfizierten MCF-7 Zelllinie wurde über eine Nickel-Sepharose Säule aufgereinigt und daraus 250 µg homogenes His-myc-EK-BSP gewonnen. Das so aufgereinigte Expressionsprodukt war partiell glykosyliert, besaß aber keine Glykosylierung an Threonin 125, das heißt dem Threonin in der BSP-Sequenz YT¹²⁵ GLAA.

Für die Glykoanalytik wurden die N-Glykane vom rekombinanten BSP (rBSP) bzw. dem Knochen-BSP enzymatisch mit dem Peptid N-Glykosidase F (PNGase F, Roche) abgetrennt. Das Enzym bewirkt eine katalytische Spaltung allen N-Glykan-Typen von den Asparaginen. Für den Verdau wurden 20 bis 200 µg BSP mit Methanol gefällt und das Fällungspellet in 1% SDS, β-Mercaptoethanol, 0,1 M BDTA 30 Minuten bei Raumtemperatur mit einem überschuß an Enzym inkubiert. Es folgte ein Verdau mit N-Glykosidase F über Nacht bei 37°C. Zum Entsalzen der N-Glykan-Lösung wurde der Verdau über eine 150 mg Carbonsäure (Carbograph SPE, Alltech) gegeben und die N-Glykane mit 25% aCN in 0,05% TFA eluiert.

Die 0-Glykane wurde mittels wasserfreier Hydrazinolyse unter eines Kits (Oglycan relase kit, Glyco) vom BSP abgspalten. Hierzu wurden etwa 200 µg salzfreies BSP 24 Studen lyophilisiert, unter Argon-Schutzgas mit 50µl Flydrazin-Reagenz versetzt, gelöst und 5 Stunden bei 60°C inkubiert. Das Hydrazin wurde unter Vakuum abgezogen. Es folgt eine Re-N-Acetylierung der N-AcetylGruppen mit Essigsäureanhydrid.

Die N- und O-Glykane wurde mit dem Fluoreszenzfarbstoft 2-aminobenzamid (Fluka) markiert und die 2-AB markierten Oligosaccharide sequenziell mit spezfischen terminalen Glykosidasen verdaut und mittels MALDI-TOF-Massenspektrometrie analysiert.

### Diskussion der Analytik

Die Aminosäuresequenz von humanem BSP enthält vier potentielle N-Glykosylierungsstellen an den Positionen 88 (NTT), 161 (NGT), 166 (NST) und 174 (NGS). Für O-Glykosylierungen ist keine vergleichbare Konsensussequenz bekannt. Alle identifizierten N-Glykan-Sturkutren waren sowohl auf dem aus Knochen isolierten BSP als auch auf dem rekombinanten EBNA-293 BSP zu finden. Unterschiede gab es jedoch im prozentualen Anteil der jeweiligen Strukturen an den gesamten N-Glykanen. So bestand der Hauptanteil der BSP-N-Glykane im Knochen aus triantennären Strukturen (58%) und in der EBNA-zelllinie aus tretraantennären Strukturen (48%).

Zur Lokalsierung der O-Gfykosylierungsstellen von rekombinantem BSP wurden die O-Glykane durch sequenziellen Verdau des Proteins mit Neuraminidase, β-Galactosidase, und β-N-Acetylhexosaminidase bis auf das core-GalNAc entfernt. Das partiell deglykosylierte Protein wurde dann durch Behandlung mit Trpysin und V8-Protease in Peptidfragment gespalten. Mittels MALDA-TOF-Massenspektrometrie wurden die Massen der Peptide bestimmt und ein Teil der Peptide mittels PSD-MALDI-TOF-Massenspektrometrie sequenziert. Mit diesem Verfahren konnten acht O-Glyknsylierungsstellen des rekombinanten BSP bestimmt werden, 5 auf dem Peptid 211-229 (TTTSP ... QVYR) und maximal drei auf dem Peptid zwischen AS 120 und AS 135 mit der Sequenz TGLAA.. Hiervon sind im rekombinanten BSP die Threonine in der Sequenz DATPGTG O-glykosyliert. Bei Knochen-BSP erfolgt eine dritte O-Glykosylierung. Bei rekombinantem BSP ist keine dritte Glykosylierungsstelle vorhanden. Vermutlich liegt diese Glykosylierungsstelle auf der TGLAA-BSP-Teilstruktur.

### Beispiel 5 - Herstellung von Anti-BSP IgY aus Eidottern

Für die Aufreinigung größerer Mengen von Anti-BSP-IgY für die Therapie und Immunszintigraphie sind verschiedene Verfahren beschrieben. Bevorzugt wird das Verfahren von Akita und Nakai benutzt (Akita E..M et al., Comparison of four purificafion methods for the production of immunoglobulin from eggs laid by hens immunized with an enterotoxigenic E. coli strain, in J Immunol Methods. 1993, 160(2), 207-14).

Für die Eiproduktion wird eine Hochleistungsrasse wie "Lohmann Weiß" oder "Lohmann Braun" mit einer Leistung von 4,5 Eiern pro Woche und einer Produktion von über 10 mg spezifischen IgY pro Dotter verwendet. Die Immunisierung erfolgt mit aus menschlichem Knochen isoliertem oder rekombinantem BSP-Andgen in Freundschen Adjuvans, wobei nach einer Grundimmunisierung mit ca. 0,1 mg BSP alle 6 Wochen Boosterinjektionen gesetzt werden. Ca. 30 % dieser Hohner reagieren normalerweise nicht auf die Immunisierung. Die Eier werden äußerlich mit Peressigsäure desinfiziert, dann aufgeschlagen und die Dotter vom Eiweiß abgetrennt. Die Dotter werden dann mit 5 bis 10 Volumina eiskaltem destilliertem Wasser zwischen pH 5 und 5,2 verquirlt und bei 2 bis 5 ° C Ober 2 bis 6 h inkubiert Dabei sedimentieren die Dottergranula, die im wesentlichen aus Lipoproteinen bestehen. Der wässrige Überstand wird dann durch Filterpapier (z.B. Whatman No. 1) klar filtriert Aus diesem Überstand können die Anti-BSP-IgY direkt über Affinitätschromatographie homogen aufgereinigt werden. An eine mit Cyanogenbromid aktivierte Sepharose-4B-Säule wird aus menschlichem Knochen oder aus Kulturüberständen von rekombinanten humanen Zelllinien isoliertes BSP chemisch kovalent gebunden. Zur Bindung von 1 g IgY ist 0,5 g immobilisiertes BSP erforderlich (kovalent an ca. 5 ml Sepharose gebunden).

Das gebundene IgY wird über einen Säuregradienten eluiert und danach die Lösung neutralisiert. Diese Lösung muß dann entsalzt und die Antikörper konzentriert werden, was im Crossflowverfahren im großen Maßstab möglich ist (z.B. Amicon Spiralfilter SY100 mit einem Ausschluß von 100.000 Dalton).

### Beispiel 6 - Isolierung von Anti-BSP IgY, das an den BSP-Faktor-H-Komplex bindet

Die geringe Reaktion der polyklonalen Hühnerantikörper mit BSP in der Knochenmatrix kann durch Selektion jener Antikörper ausgeschaltet werden, die mit BSP im Komplex mit Faktor H reagieren. Dazu wird entweder Faktor H oder aus Knochen isoliertes oder gentechnisch hergestelltes BSP an Cyanogenbromid-aktivierte Sepharose 4 B chemisch kovalent gebunden und danach soviel BSP bzw. Faktor H auf die Säule aufgetragen und gebunden, dass sämtliche Liganden in der Matrix mit dem Partner komplexiert sind. Filtrierter Dotterextrakt wird dann auf dieser Affinitätssäule aufgetragen und wie in Beispiel 4 wird nun jene Antikörperfraktion gewonnen, die spezifisch an das freie Epitop im BSP-Faktor-H Komplex bindet

### Beispiel 7 - Herstellung von humanen Anti-BSP Antikörpern in transgenen Hühnern

Anti-BSP-IgY weisen in der Humantherapie bzw. Diagnostik einige Schwächen auf. So sind Nebenwirkungen wie Frerndeiweißreaktionen zu erwarten und die biologische Halbwertszeit beträgt im Vergleich zu humanen Antikörpern nur 12 bis 24 Stunden. IgY aktiviert auch nicht das Komplementsystem.

Humane Antikörper gegen BSP können in speziellen transgenen Hühnern hergestellt werden, in denen durch Gene-Targeting die konstante Region für aviäres Immunglobulin in den für die Antikörperbildung verantwortlichen Genen durch die konstante Region für humanes Immunglobulin ausgetauscht wurde. Geeignete Huhnstammzellen und Vektorsysteme sind in den US-Patenten 5,340,740, Nr. 5,656,479 und Nr. 5,464,764 beschrieben. Nach Immunisierung mit BSP reagieren solche Hühner mit der Produktion von humanen Antikörpern im Ei.

### Beispiel 8 - Immunbiotanalyse der Expression von BSP in menschlichen Brustkrebszellinien

Die Tumorzeilinien MDA-MB-231 (Brustkrebszellinie, Östrogenrezeptor-negativ), MCF-7 (Brustkrebszellinie, Östrogen rezeptor-positiv) und T-47-D (Brustkrebszellinie, Östrogenrezeptor-positiv) wurden mit Immunpräzipitationspuffer extrahiert und BSP mit dem polyklonalen Antikörpergemisch A0001 aus Kaninchen gegen humanes BSP gefällt Die Präzipitate wurden nach Denaturierung auf SDS-Gelen aufgetragen, die Elektrophorese durchgeführt und die Proteine auf Nitrozellulosemembranen übertragen. Danach erfolgte eine Immunfärbung mit dem anti-BSP-Kaninchenantiserum A001 und einem monoklonalen Maus-Anti-BSP-Antikörper (BSP 1.2), wobei als Zweitantikörper Peroxidasekonjugate von Antikörper der Ziege gegen Kaninchen IgG und gegen Maus IgG verwendet wurden. Auf beiden Blots A und B war die Bande des immunpräzipitieren BSP bei 70.000 Dalton deutlich zu erkennen.

Um die Anwesenheit bzw. Abwesenheit von BSP auf der Zelloberfläche von Tumorzellen zu zeigen, wurden die Zelloberflächen der Brustkrebszellinien MDA-MB-231 und MCF-7 biotinyliert, mit Immunpräzipitationspuffer extrahiert und BSP mit dem polyklonalen Antikörpergemisch A0001 aus Kaninchen gegen humanes BSP gefällt Die Präzipitate wurden nach Denaturierung auf SDS-Gelen aufgetragen, die Elektrophorese durchgeführt und die Proteine auf eine Nitrozellulosemembran übertragen. Biotinylierte Proteine auf dieser Membran wurden dann mit einem Konjugat aus Peroxidase und Streptavidin mit dem ECL-System (Amersham) nachgewiesen.

### Beispiel 8a - Expression von BSP in und auf Brustkrebszellinien

Menschliche Brustkrebszellen der Linien T-47-D und MDA-MB-231 wurden mit und ohne vorherige Permeabilisierung mit einem anti-Schweine-BSP-Antikörper aus Kaninchen und einem mit Fluorescein konjugierten anti-Kaninchenantikörper der Ziege immunfluoreszent markiert Fluoreszent markiertes BSP ist in beiden Zeilinien nach Permeabilisierung zu erkennen Nur auf den T-47-D Zellen ließ sich auch ohne Permeabilisierung BSP über Immunfluoreszenz nachweisen.

### Beispiel 9 - Nachweis der BSP-Expression in Tumorzellen über RT-PCR

Aus den Tumorzellinien MDA-MB-231 (Brustkrebszellinie, Östrogenrezeptor-negativ, MCF-7 (Brustkrebszellinie, Östrogenrezeptor-positiv) und T-47-D (Brustkrebszellinie, Östrogenrezeptor-positiv) und humanen Fibroblasten (HGF) als Kontrolizellen wurde mRNA isoliert, durch Reverse Transkriptase die komplementäre cDNA hergestellt und die BSP-cDNA durch PCR mit BSP-spezifischen Primem amplifiziert. Die Expression von BSP-mRNA war in der Brustkrebszellinie MCF-7 besonders hoch, bei den MDA-MB-231 und T-47-D Zellen gering und bei der Kontrollzellinie nicht nachweisbar.

### Beispiel 10 - Herstellung von humanisierten monoklanaten Antikörpern

Der monoklonale Antikörper BSP 1.2 kann aufgrund seiner spezifischen Bindung an Tumor-BSP für die Therapie von Primärtumoren und Metastasen eingesetzt werden. Dabei bindet der Antikörper an BSP auf der Zelloberfläche bestimmter. Tumorzellen und stimuliert das Immunsystem zur Zerstörung dieser Zellen z.B. über die Akfivierung der Komplementkaskade. Ähnlich lassen sich auch die polyklonalen oder monoklonale Anti-BSP IgY für die Therapie einsetzen. Beim Einsatz dieser Antikörper reagiert das menschliche Immunsystem mit der Bildung von eigenen Antikörper - humane Anti-Maus-IgG Antikörper (HAMAs) bzw. humane Anti-Huhn-IgY Antikörper (HACAs). HAMAs und HACAs können eine Immunantwort des Organismus auf das Tumorantigen induzieren und verstärken. Bei der Bestimmung von Tumormarkern entstehen jedoch Interferenzen mit den HAMAs und HACAs, die die in vitro Meßmethode stören. Auf diese Weise kommt es zu falsch hohen Meßwerten für Tumormarker.

Deshalb eignen sich für die Therapie und Immunszintigraphie besonders humanisierte monoklonale Antikörper. Mehrere Verfahren sind beschrieben, wie man aus den. Hybridomazelllinien, welche monoklonale Anti-BSP Antikörper produzieren, entsprechende humanisierte Antikörper ableitet.

### Beispiel 11- Konjugate von Anti-BSP Antikörpern mit Zellgiften und Radioisotopen

In einer weiteren Anwendung der Erfindung können Zellgifte und Radioisotope chemisch kovalent mit den Anti-BSP Antikörper oder deren Fab-Fragmente verbunden werden. Mit Radioisotopen wie Jod 125 oder Jod 131 markierte Antikörper eignen sich bei Applikation geringer Mengen zur Tumorlokalisation Ober die Immunszintigraphie und bei Applikation großer Mengen zur direkten Zerstörung der Tumore. Solche chemischen Konjugate können zum Beispiel durch Jodierung der Antikörper mit Jod 125 oder 131 hergestellt werden (Garvey, J.S et al., Methods in Immunology. 3rd ed., W.A.Benjamin Publ., 1977, 171-182). Eine Übersicht über geeignete Verfahren zur Radioimmuntherapie und immunszintigraphie findet sich bei Vuillez, *Radioimmunotargeting: diagnosis and therapeutic use*, *in* Bull Cancer. 2000, 87(11), 813-27.

### Beispiel 12 - Therapie von Tumoren mit Expression von BSP auf der Zelloberfläche

Es wurde an Biopsiematerial zunächst festgestellt, ob BSP auf der Oberfläche der Tumorzellen exprimiert wird. Patienten, bei denen BSP auf der Oberfläche der Tumorzellen nachweisbar ist, kommen für eine Therapie mit Anti-BSP Antikörpern des Huhns, der Maus, den entsprechenden humanisierten Antikörpern und mit Konjugaten dieser Antikörper mit Zellgiften oder Radioisotopen in Frage.

Die Behandlung von Tumoren mit therapeutischen Antikörpern, die gegen auf Zelloberflächen exprimierte Tumormarker gerichtet sind, ist Stand der Technik. So kann mit dem humanisierten Antikörper Herceptin gegen den Rezeptor für den humanen Epithelwachstumsfaktor Brustkrebs seibst in der metastasierten Form bei ca. 25 % der Betroffenen erfolgreich therapiert werden (Hotaling TE et al., The humanized anti-HER2 antibody rhuMAb HER2 mediates antibody dependent cell-mediated cytotoxicity via FcgR III [abstract]. Proc Annu Meet Am Assoc Cancer Res 1996; 37:47; Pegram MD et al., Antibody dependent cell-mediated cytotoxicity in breast cancer patients in Phase III clinical trials of a humanized anti-HER2 antibody [abstract]. Proc Am Assoc Cancer Res 1997; 38:602.

Ähnlich wie bei Herceptin kann der entsprechende Anti-BSP Antikörper als Infusion appliziert werden, z.B. als 90 Minuten Infusion bei der Erstapplikation und später als 30 Minuten Infusion. Die Häufigkeit der Infusionen und die Menge der Antikörper richten sich nach der Halbwertszeit der Antikörper im Blut (ca. 6 Tage bei einem humanisierten Antikörper und weniger als 24 Stunden bei einem Hühnerantikörper und dem Körpergewicht.

### Beispiel 13 - Therapie von Tumoren durch Neutralisierung von freiem nicht an Zellen gebundenen BSP und des BSP-Faktor-H Komplexes

Mit den oben beschriebenen Verfahren wird festgestellt, ob die Tumorzellen des Patienten BSP exprimieren, das nicht auf der Zelloberfläche nachgewiesen werden kann. Bei diesen Tumoren kann davon ausgegangen werden, dass die Zellen BSP in das Blut oder die Gewebsffüssigkeit abgeben und z.B. zur Bindung von Faktor H zur Inaktivierung des alternativen Wegs der Komplementkaskade oder zur Einwanderung in Knochengewebe nutzen. Ein weiterer möglicher Indikator für diesen Tumortyp sind erhöhte Konzentrationen des BSP im Blutserum (> 20 ng / mL Serum). In diesen Fällen können Anti-BSP Antikörper zur Neutralisierung des freien bzw. mit Faktor H komplexierten Tumor-BSP eingesetzt werden. Die Dosis kann dabei auf die Menge des frei im Serum und in der Gewebsflüssigkeit vorhandenen BSP abgestimmt werden. Für die Therapie kommen Anti-BSP Antikörper aus dem Huhn, der Maus und humanisierte Anti-BSP Antikörper in Frage, die das freie BSP-Epitop im Komplex mit Faktor H erkennen können. Auch in Frage kommen Fab-Fragmente dieser Antikörper, die nach einem Standardverfahren durch proteolytischen Verdau präpariert werden können (Garvey, J.S et al., Methods in Immunology. 3rd ed., W.A.Benjamin Publ., 1977, 256 - 266). Auch gentechnisch hergestellte aus den obigen Anti-BSP Antikörpern abgeleitete Fab Fragmente kommen für eine solche Therapie in Frage.

Die Erfindung stellt somit Antikörper bereit gegen das humane Bone-Sialoprotein (hBSP), die spezifisch nur Epitope auf hBSP von Tumorzellen binden, denn Tumor-hBSP enthält keine posttranslationale O-Glykosylierung im Bereich der Aminosäuren 120 bis 135 (SWISSPROT: SIAL_HUMAN, Acc.No. P21815, beinhaltend die Aminosäuren TGLAA. Anders das normale hBSP aus Knochen. Die Antikörper können tumorgenes Serum-hBSP im Komplex mit dem Komplementfaktor-H erkennen und bilden somit ein diagnostisch und therapeutisches werlvolles Instrument.

### Beispiel 14 - RIA-Messreihen an Seren von Dialyse- und Prostatapatienten

Die erfindungsgemäßen Antikörpern wurden als Fängerantikörper in ELISAs eingesetzt und in vergleichenden Messreihen die Menge an BSP im Serum von Dialysepatienten ("normales" BSP) und Prostatapatienten mit vereinzelt stark erhöhten BSP.Werten tumorgenen Ursprungs untersucht.

Die Serum-Konzentrationen von humanem Bone-Sialoprotein wurden untersucht mit erfindungsgemäßen polyklonalen Antikörpern aus Huhn, die auch an Epitope binden, die auf humanem Bone-Sialoprotein aus Tumorzellen oder krankheitsbedingt stark belasteten Geweben vorhanden sind, das heißt auf einem humanem BSP, dessen posttranslationale Glykosylierung im Bereich der Aminosäuren 120 bis 135, beinhaltend die Aminosäuren TGLAA, gegenüber normalem Bone-Sialoprotein aus gesunden Knochen verändert oder unvollständig ist. In der Regel wurden 100 µL der AntikörperLösung mit 125I-markiertem Bone-Sialoprotein gemischt Nach 24-stündiger inkubation bei 4°C wurden 100 µL Zweitantikörper-Lösung zugesetzt (Esel-Anti-Huhn-IgG) und nach 2 Std. Inkubation bei 4°C die Reaktionsmischung bei 2000 G zentrifugiert und der Überstand abgenommen. Nach einem Waschen mit 250 µL in PBS und einer Nachzentrifugation (10 Minuten bei 2000 G) wurde erneut der Überstand abgenommen und die Radioaktivität des Pellets nach Zusatz einer Zahlflüssigkeit für 1 Minute mit einem Gamma-Zähler bestimmt.

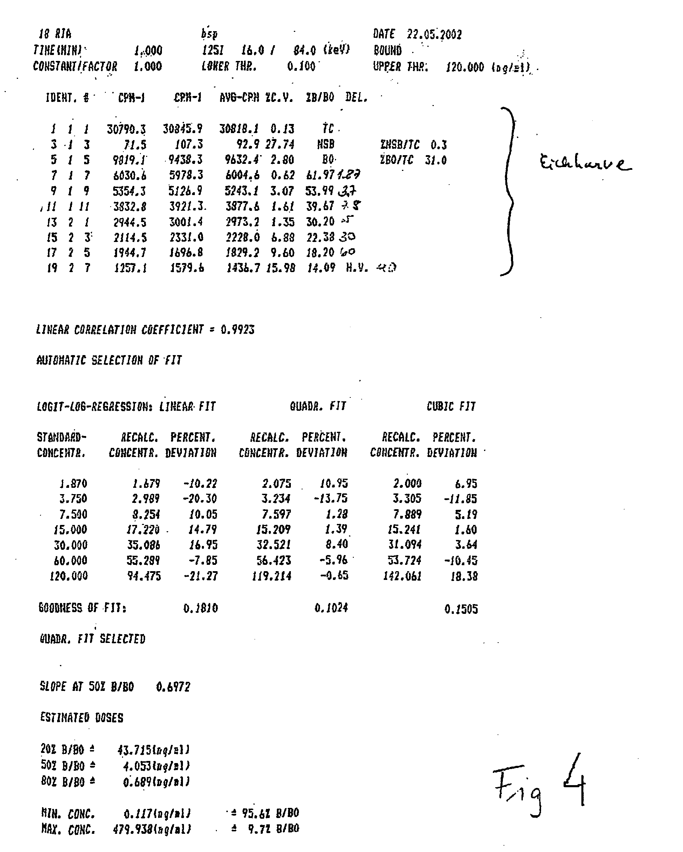

Die notwendige Eichkurve von in üblicher Weise erstellt und zwar mithilfe eines4-Parameter Kurvenalgorithmus. Die Standardwerte enthielten in der Regel 0, 1.9, 3.8, 7.5, 15, 30, 60 und 120 µg/L BSP Die Bewertung der analytischen Genauigkeit durch Zusatz von Kalibrator zu den Patientenproben ergab im Mittel eine Wiederfindung im Bereich von Ober 99%. Der Computerausdruck für eine Mustereichkurve ist in Fig. 4 dargestellt.

Die nachstehenden tabellarischen Messreihen zeigen, dass mithilfe der erfindungsgemäßen Antikörper zuverlässig tumorgenes und unverändertes BSP im Serum von Patienten quantitativ bestimmt werden kann, und zwar in Gegenwart von Faktor-H und unabhängig vom jeweiligen Krankheitsbild der Patienten. Eine mühsame und fehlerbelastete Auftrennung der Serumproteinen wie Faktor H von BSP ist nicht erforderlich. Insbesondere kann BSP aus Tumorzellen zuverlässig bestimmt werden, was erhebliche Fortschritte in der Prognostik bzw. der Charakterisierung des Primärtumors und eventueller Metastasen hiervon mit sich bringt

**TABELLE 2- Messreihe von Serum von Patienten mit Prostatacarcinom.**

| **Kasten 1-4** | **ID-Nr. Sialo** | **Proben Nr.** | **Code** | **Diagn** | **Proben-volumen** | **Sialoprotein (ig/L)** |
|---|---|---|---|---|---|---|
| 1 | 1 | 182 | E096 | 7 | | 81,2 |
| 1 | 2 | 183 | E162 | 7 | | 479,0 |
| 1 | 3 | 184 | E278 | 7 | | 28.0 |
| 1 | 4 | 185 | E476 | 7 | | 10,8 |
| 1 | 5 | 186 | E416 | 7 | | 38,5 |
| 1 | 6 | 187 | E560 | 7 | | 3,9 |
| 1 | 7 | 188 | H180 | 7 | | 30,2 |
| 1 | 8 | 189 | E653 | 7 | | 344,7 |
| 1 | 9 | 190 | D268 | 7 | | 112,3 |
| 1 | 10 | 191 | E527 | 7 | | 17,4 |
| 1 | 11 | 192 | E524 | 7 | | 18,5 |
| 1 | .12 | 193 | E441 | 7 | | 72,2 |
| 1 | 13 | 194 | H096 | 7 | | 251,0 |
| 1 | 14 | 195 | E824 | 7 | | 33,4 |
| 1 | 15 | 196 | E971 | 7 | | >Messbereich |
| 1 | 16 | 197 | D411 | 7 | | 10,9 |
| 1 | 17 | 198 | H601 | 7 | | 41,1 |
| 1 | 18 | 199 | H567 | 7 | 130iL | >Messbereich |
| 1 | 19 | 272 | H280 | 7 | | 31,5 |
| 1 | 20 | 273 | H316 | 7 | | |
| 1 | 21 | 274 | H420 | 7 | | 238,9 |
| 1 | 22 | 275 | I006 | 7 | | 17,9 |
| 1 | 23 | 276 | I007 | 7 | | 88,0 |
| 1 | 24 | 277 | I033 | 7 | | 27,2 |
| 1 | 25 | 278 | I084 | 7 | | 80,2 |
| 1 | 26 | 279 | I093 | 7 | | 29,1 |
| 1 | 27 | 280 | I142 | 7 | | 447,0 |
| 1 | 28 | 281 | 9407(Mü) | 7 | | 252,0 |
| 1 | 29 | 282 | 9245(Mü) | 7 | | 37,3 |
| 1 | 30 | 283 | 8940(Mü) | 7 | 250iL | 102,9 |
| 1 | 31 | 284 | 8779(Mü) | 7 | | |
| 1 | 32 | 285 | 8712(Mü) | 7 | | 11,9 |
| 1 | 33 | 286 | 8689(Mü) | 7 | | 7,4 |
| 1 | 34 | 287 | 8400(Mü) | 7 | | 32,7 |
| 1 | 35 | 288 | 8388(Mü) | 7 | | 30,8 |
| 1 | 36 | 289 | 8279(Mü) | 7 | | |
| 1 | 37 | 290 | H125 | 7 | | >Messbereich |
| 1 | 38 | 291 | H172 | 7 | | 9,0 |
| 1 | 39 | 292 | H180 | 7 | - | |
| 1 | 40 | 293 | H487 | 7 | | 5,9. |
| 1 | 41 | 104 | F968 | 4 | | 3,2 |
| 1 | 42 | 105 | F965 | 4 | | 76,7 |
| 1 | 43 | 106 | F923 | 4 | | 8,4 |
| 1 | 44 | 107 | F908 | 4 | | 11,1 |
| 1 | 45 | 108 | F821 | 4 | | 17,7 |
| 1 | 46 | 109 | F836 | 4 | | 3,5 |
| 1 | 47 | 110 | F342 | 4 | | 3,0 |
| 1 | 48 | 111 | F214 | 4 | | 8,4 |
| 1 | 49 | 112 | G843 | 4 | | 6,7 |
| 1 | 50 | 113 | G777 | 4 | | 10,9 |
| 1 | 51 | 114 | G753 | 4 | | 6,8 |
| 1 | 52 | 115 | G381 | 4 | | 3,1 |
| 1 | 53 | 116 | G984 | 4 | | 32,1 |
| 1 | 54 | 117 | G891 | 4 | | 17,0 |
| 1 | 55 | 118 | F196 | 4 | | 3,4 |
| 1 | 56 | 119 | F183 | 4 | | 6,9 |
| 1 | 57 | 120 | G010 | 4 | | 20,6 |
| 1 | 58 | 121 | F453 | 4 | | 6,0 |
| 1 | 59 | 122 | F640 | 4 | | 2,6 |
| 1 | 60 | 123 | G462 | 4 | | 10,7 |
| 1 | 61 | 124 | F423 | 4 | | 4,7 |
| 1 | 62 | 125 | F360 | 4 | | 3,0 |
| 1 | 63 | 126 | F875 | 4 | | 7,5 |
| 2 | 64 | 127 | G798 | 4 | | 8,9 |
| 2 | 65 | 128 | G786 | 4 | | 4,9 |
| 2 | 66 | 129 | G384 | 4 | | 8,2 |
| 2 | 67 | 130 | F529 | 4 | | 7,1 |
| 2 | 68 | 131 | E405 | 4 | | 1,3 |
| 2 | 69 | 132 | E419 | 4 | | |
| 2 | 70 | 133 | E274 | 4 | | 17,2 |
| 2 | 71 | 134 | E545 | 4 | | 6,3 |
| 2 | 72 | 135 | E166 | 4 | | 14,5 |
| 2 | 73 | 136 | F601 | 4 | | 8,0 |
| 2 | 74 | 137 | G432 | 4 | | 5,3 |
| 2 | 75 | 138 | F264 | 4 | | 4,5 |
| 2 | 76 | 139 | D297 | 4 | | |
| 2 | 77 | 140 | E355 | 4 | 1801L | 8,9 |
| 2 | 78 | 141 | E950 | 4 | | 10,0 |
| 2 | 79 | 142 | E391 | 4 | | 8,3 |
| 2 | 80 | 69 | F053 | 3 | | 11,0 |
| 2 | 81 | 70 | F177 | 3 | | 6,5 |
| 2 | 82 | 71 | F068 | 3 | | 8,0 |
| 2 | 83 | 72 | F237 | 3 | | 3,6 |
| 2 | 84 | 73 | F255 | 3 | | 7,5 |
| 2 | 85 | 74 | F378 | 3 | | 2,1 |
| 2 | 86 | 75 | F471 | 3 | | 7,5 |
| 2 | 87 | 76 | F484 | 3 | | 6,0 |
| 2 | 88 | 77 | F555 | 3 | | 12.5 |
| 2 | 89 | 78 | F281 | 3 | | 7,0 |
| | 90 | 79 | F287 | 3 | | 4,0 |
| 2 | 91 | 80 | F372 | 3 | | 3,8 |
| 2 | 92 | 81 | F496 | 3 | | |
| 2 | 93 | 82 | F536 | 3 | | 4,2 |
| 2 | 94 | 83 | F561 | 3 | | 9,9 |
| 2 | 95 | 84 | F573 | 3 | | |
| 2 | 96 | 85 | F604 | 3 | | 6,6 |
| 2 | 97 | 86 | F651 | 3 | | 5,4 |
| 2 | 98 | 87 | F689 | 3 | | 1,7 |
| 2 | 99 | 88 | F695 | 3 | | 5,7 |
| 2 | 100 | 89 | F728 | 3 | | 8,8 |
| 2 | 101 | 90 | F785 | 3 | 200iL | 3,0 |
| 2 | 102 | 91 | F797 | 3 | | 5,3 |
| 2 | 103 | 92 | F824 | 3 | | 3,3 |
| 2 | 104 | 93 | F005 | 3 | 220iL | 2,6 |
| 2 | 105 | 94 | F017 | 3 | | 3,0 |
| 2 | 106 | 95 | F866 | 3 | | 6,3 |
| 2 | 107 | 96 | F881 | 3 | | |
| 2 | 108 | 97 | F890 | 3 | | 6,7 |
| | 109 | 98 | F935 | 3 | | 5,7 |
| 2 | 110 | 99 | F944 | 3 | | 6,7 |
| 2 | 111 | 100 | F947 | 3 | | 8,0 |
| 2 | 112 | 101 | F950 | 3 | | |
| 2 | 113 | 102 | F983 | 3 | | 8,9 |
| 2 | 114 | 103 | F986 | 3 | | 5,7 |
| 2 | 115 | 1 | H128 | 1 | | 5,2 |
| 2 | 116 | 2 | H073 | 1 | | 8,9 |
| 2 | 117 | 3 | H072 | 1 | | 18,8 |
| 2 | 118 | 4 | H131 | 1 | | 6,9 |
| 2 | 119 | 5 | H082 | 1 | | 5,3 |
| 2 | 120 | 6 | H052 | 1 | | 3,2 |
| 2 | 121 | 7 | H057 | 1 | 120iL | 5,2 |
| 3 | 122 | 8 | H060 | 1 | | 2,7 |
| 3 | 123 | 9 | H061 | 1 | | 2,7 |
| 3 | 124 | 10 | H062 | 1 | | 2,0 |
| 3 | 125 | 11 | H063 | 1 | | 3,0 |
| 3 | 126 | 12 | H065 | 1 | | 4,3 |
| 3 | 127 | 13 | H067 | 1 | | 3,9 |
| 3 | 128 | 14 | H070 | 1 | 120iL | 4,3 |
| 3 | 129 | 15 | H097 | 1 | | 3,8 |
| 3 | 130 | 16 | H116 | 1 | 220iL | 11,0 |
| 3 | 131 | 17 | H134 | 1 | | 3,9 |
| 3 | 132 | 18 | H139 | 1 | | 3,7 |
| 3 | 133 | 19 | H140 | 1 | | 12,1 |
| 3 | 134 | 20 | H143 | 1 | | 7,2 |
| 3 | 135 | 21 | H163 | 1 | | 5,1 |
| 3 | 136 | 22 | H051 | 1 | | 3,1 |
| 3 | 137 | 23 | H244 | 1 | | 6,7 |
| 3 | 138 | 24 | H271 | 1 | | 2,6 |
| 3 | 139 | 25 | H006 | 1 | | 4,7 |
| 3 | 140 | 26 | H250 | 1 | | 3,5 |
| 3 | 141 | 27 | H389 | 1 | | 260,0 |
| 3 | 142 | 28 | H302 | 1 | | 2,9 |
| 3 | 143 | 29 | H336 | 1 | | 8,0 |
| 3 | 144 | 30 | H004 | 1 | | 11,8 |
| 3 | 145 | 31 | G619 | 1 | - | |
| | 146 | 32 | D749 | 1 | | 8,7 |
| 3 | 147 | 33 | D761 | 1 | | 6,6 |
| 3 | 148 | 34 | 1 | 1 | | 4,2 |
| 3 | 149 | 35 | 16 | 1 | | 8,5 |
| 3 | 150 | 36 | G969 | 1 | | 27,2 |
| 3 | 151 | 148 | E032 | 6 | | 9,8 |
| 3 | 152 | 149 | E938 | 6 | | 9,4 |
| 3 | 153 | 150 | E674 | 6 | 210iL | 11,5 |
| 3 | 154 | 151 | E890 | 6 | | 10,5 |
| 3 | 155 | 152 | E082 | 6 | | 27,7 |
| 3 | 156 | 153 | E395 | 6 | 210iL | 13,9 |
| 3 | 157 | 154 | E668 | 6 | | 27,9 |
| 3 | 158 | 155 | E242 | 6 | | |
| 3 | 159 | 156 | G320 | 6 | | 7,6 |
| 3 | 160 | 157 | G248 | 6 | | 9,9 |
| 3 | 161 | 158 | G055 | 6 | | 14,5 |
| 3 | 162 | 159 | G034 | 6 | | 5,8 |
| 3 | 163 | 160 | G804 | 6 | | 7,2 |
| 3 | 164 | 161 | G720 | 6 | | 6,2 |
| 3 | 165 | 162 | G774 | 6 | | 10,6 |
| | 166 | 163 | G699 | 6 | | 2,7 |
| 3 | 167 | 164 | G672 | 6 | | 9,0 |
| 3 | 168 | | F776 | 6 | | 15,5 |
| 3 | 169 | 166 | F869 | 6 | | 4,3 |
| 3 | 170 | 167 | H169 | 6 | | 8,9 |
| 3 | 171 | | F199 | 6 | | 13,2 |
| 3 | 172 | 169 | F246 | 6 | | 46,0 |
| 3 | 173 | 170 | F502 | 6 | | 7,4 |
| 3 | 174 | 171 | E806 | 6 | | 7,5 |
| 3 | 175 | 172 | E929 | 6 | | 18,2 |
| 3 | 176 | 173 | E494 | 6 | | 6,7 |
| 3 | 177 | 174 | E758 | 6 | | 6,8 |
| 3 | 178 | 175 | E692 | 6 | | 15,4 |
| 3 | 179 | 176 | G025 | 6 | | 19,3 |
| 3 | 180 | 177 | G723 | 6 | | 16,0 |
| 3 | 181 | 178 | F508 | 6 | | 32,7 |
| 4 | 82 | 179 | G227 | 6 | | 8,1 |
| 4 | 183 | 180 | H261 | 6 | | 23,8 |
| 4 | 184 | 181 | H314 | 6 | | 28,0 |
| 4 | 185 | 294 | H599 | 7 | - | |
| 4 | 186 | 295 | H601 | 7 | - | |
| 4 | 187 | 296 | H737 | 7 | | 7,6 |
| 4 | 188 | 297 | H955 | 7 | | 14,6 |
| 4 | 189 | 298 | H980 | 7 | | 10,0 |
| 4 | 190 | 299 | 1010 | 7 | | 3,7 |
| 4 | 191 | 300 | 1282 | 7 | | 18,0 |
| 4 | 192 | 301 | 9742(Mü) | 7 | | 42,0 |
| 4 | 193 | 302 | 9773(Mü) | 7 | | 22,3 |
| 4 | 194 | 303 | 9788(Mü) | 7 | | 15,7 |

Die Diagnostikziffern stehen für:
1 = GesMänner
3 = BPH
4 = PCa pN0 M0
6 = PCa pN1 M0
7 = PCa pN1 M1 ossär

Die pathomorphologische Klassifikation der Carcinome erfolgte nach Van Nuys. M steht für Metastasen.

**TABELLE 3 - Bestimmung von BSP im Serum von Dialysepatienten.**

| **Proben-Nr.** | **Patient-Code** | **BSP ng/ml Serum** | **Bemerkung: Gerätesequenz** |
|---|---|---|---|
| L50 | G**.E. | 18,8 | 10 Serum>AxSym1(alt):B/10 |
| L69 | H**.R. | 10.1 | 10Serum>AxSym1(alt):F/07 |
| L85 | W**.D | 77,3 | 10 Serum>HITACHI 917:5006/1 |
| L131 | K**.E. | 17,1 | 10 Serum>AxSym1(alt):A/09 |
| L13 | B**.M. | 36,6 | 10 Serum>AxSym1 (alt):A/04 |
| L14 | H**.L. | 6,6 | 10 Serum>AxSym1 (alt):C/04 |
| L15 | P**.B. | 8,1 | 10 Serum>AxSym1(alt):C/02 |
| L16 | S**.I. | 10,4 | 10 Serum>AxSym1(alt):E/09 |
| L24 | H**.A. | 28,1 | 10 Serum>AxSym1(alt):B/04 |
| L38 | S**.M. | 3,1 | 10 Serum>AxSym1(alt):A/03 |
| L41 | K**.C, | 12,5 | 10 Serum>AxSym1(alt):B/02 |
| L50 | H**.L. | 31.9 | 10 Serum>AxSym1(alt)::C/03 |
| L77 | W**.P. | 7,8 | 10 Serum>AxSym1(alt):A/04 |
| L64 | D**.K. | 46,9 | 10 Serum>AxSymt(alt):D/08 |
| L88 | M**.H. | 26,5 | 10 Serum>AxSym1(alt):A/01 |
| L134 | S**.M. | 4,2 | 10 Serum>AxSym1(alt):E/04 |
| L73 | H**.K. | 5,2 | 10 Serum>AxSym1(alt):D/01 |
| L75 | L**.I. | 5,5 | 10 Serum>AxSym1(alt):B/05 |
| L78 | Z**.U. | 15,2 | 10 Serum>AxSym1(alt):A/01 |
| L79 | P**.B. | 3,2 | 10 Serum>AxSym1(alt):B/01 |
| L82 | G**.B. | 29,7 | 10 Serum>AxSym1(alt):A/02 |
| L85 | S**.I. | 18,0 | 10 Serum>AxSym1(alt):E/04 |
| L91 | K**.H. | 12,4 | 10 Serum>AxSym1(alt):C/07 |
| L93 | S**.G. | 17,0 | 10 Serum>AxSym1 (alt):A/02 |
| L96 | S***.M. | 8,9 | 10 Serum>AxSym1(alt):A/07 |
| L113 | S**.G. | 18,9 | 10 Serum>AxSym1(alt):A/04 |
| L114 | R**.H. | 22,7 | 10 Serum>AxSym1(alt):A/05 |
| L117 | R**.A. | 16,0 | 10 Serum>AxSym1(alt):A/09 |
| L53 | L**.E. | 44,4 | 10 Serum>AxSym1(alt):A/08 |
| L71 | M**.M. | 11,4 | 10 Serum>AxSym1(alt)::B/04 |
| L94 | W**.E. | 19,0 | 10 Serum>AxSym1(alt):B/04 |
| L10 | S**.L. | 12,3 | 10 Serum>AxSym1(alt):E/03 |
| L12 | N**.J. | 5,0 | 10 Serum>AXSym1(alt):D/02 |
| L13 | C**.F: | 7,0 | 10 Serum>AxSym1 (alt):D/04 |
| L17 | L**.I. | 6,7 | 10 Serum>AxSym1(alt):A/05 |
| L18 | S**.R. | 8,7 | 10 Serum>AxSym1(alt):A/06 |
| L19 | L**.E. | 11,0 | 10 Serum>AxSym1(alt): A/09 |
| L24 | H**.F. | 18,6 | 10 Serum>AxSym1(alt):A/05 |
| L43 | H**.L. | 17,8 | 10 Serum>AxSym1(alt):B/06 |
| L45 | S**.C. | 3,1 | 10 Serum>AxSym1(alt):8/07 |
| L1 | S**.W. | 21,5 | 10 Serum>HITACHI 917:7002/1 |
| L2 | S**M. | 14,2 | 10 Serum>HITACHI 917:7020/2 |
| L3 | S**.A. | 11,8 | 10 Serum>AxSym1(alt):A/02 |
| L4 | D**.J. | 14,4 | 10 Serum>AxSym1 (alt):A/01 |
| L5 | G**.H. | 9,0 | 10 Serum>Axsym1 (alt);A/02 |
| L6 | B**.U. | 4,7 | 10 Serum>AxSym1(alt):A/03 |
| L7 | S**.L | 8,2 | 10 Serum>AxSym1 (alt):A/01 |
| L8 | H**.E. | 15,3 | 10 Serum>AxSym1(alt):A/04 |
| L9 | N**.H. | 7,1 | 10 Serum>AxSym1(alt):E/03 |

Ein erhöhter BSP-Wert im Serum (>20 - 25 µg/L) deutet hier darauf hin, dass eine pathologische Veränderung der Knochenstruktur bzw. ein erhöhter Knochenstoffwechsel vorliegt

## Patentansprüche

1. Antikörper oder eine Mehrzahl von Antikörpern gegen humanes Bone-Sialoprotein (BSP), **dadurch gekennzeichnet, dass** die Antikörper Epitope binden, die auf humanem Bone-Sialoprotein aus Tumorzellen vorhanden sind und zwar im Bereich der Aminosäuren 120 bis 135 des humanen Bone-Sialoproteins (SWISSPROT: SIALJHUMAN, Acc.No. P21815), welche die Aminosäuren TGLAA enthalten und deren posttranslationale Glykosilierung gegenüber normalem Bone-Sialoprotein aus Knochen verändert oder unvollständig ist.

2. Antikörper nach Anspruch 1, erzeugt mit einem chemisch oder natürlich in der Glykosylierung verändertem Bone-Sialoprotein als Antigen.

3. Antikörper nach Anspruch 1 oder 2, erzeugt mit einem Bone-Sialoprotein aus Tumorzellen als Antigen.

4. Antikörper nach Anspruch 3, wobei das in der Glykosylierung veränderte Bone-Sialoprotein gentechnisch in Tumorzellen hergestellt wird.

5. Antikörper nach Anspruch 1, erzeugt gegen ein peptidisches Antigen, umfassend die Aminosäuresequenz TGLAA oder YTGLAA, gegebenenfalls gekoppelt an ein Trägerprotein.

6. Antikörper nach Anspruch 2, wobei in der Glykosylierung verändertes Bone-Sialoprotein aus Knochenmaterial verwendet wird, dessen Spender nicht zur normalen Glykosylierung von Knochenproteinen fähig war.

7. Antikörper nach einem der Ansprüche 1 bis 6, die IgY-Antikörper aus Huhn sind.

8. Antikörper nach einem der Ansprüche 1 bis 6, die human oder humanisiert sind.

9. Verfahren zur Bestimmung von Bone-Sialoprotein aus Tumorzellen in Körperflüssigkeiten, insbesondere in Serum, wobei Antikörper nach einem der Ansprüche 1 bis 8 verwendet werden.

10. Verfahren nach Anspruch 9 zur Diagnostik und Prognostik von Knochenmetastasen,

11. Verwendung der Antikörper nach einem der Ansprüche 1 bis 8 zur Herstellung eines Medikaments.

12. Pharmazeutische Zusammensetzung, die als Wirksubstanz Antikörper nach einem der Ansprüche 1 bis 8 enthält.

13. Pharmazeutische Zusammensetzung nach Anspruch 12 zur Prophylaxe und Therapie von Knochenmetastasen.

14. Verwendung der Antikörper nach einem der Ansprüche 1 bis 8 als Targeting-Mittel zur Herstellung eines Diagnostikmittels oder Medikaments.

## Claims

1. Antibodies or a plurality of antibodies against human bone sialoprotein (BSP), **characterized in that** the antibodies bind epitopes which are located on human bone sialoprotein from tumour cells in the region of amino acids 120 to 135 of human bone sialoprotein (SWISSPROT: SIALJHUMAN, Acc. No. P21815), comprising amino acids TGLAA, and which posttranslational glycosylation is modified or incomplete compared to normal bone sialoprotein from bones.

2. Antibodies in accordance with claim 1, produced with a bone sialoprotein as antigen, which is chemically or naturally modified in its glycosylation.

3. Antibodies in accordance with claim 1 or 2, produced with a bone sialoprotein from tumour cells as antigen.

4. Antibody in accordance with claim 3, wherein the bone sialoprotein which is modified in its glycosylation is produced genetically in tumour cells.

5. Antibody in accordance with claim 1, produced with a peptidic antigen, comprising the amino acid sequence TGLAA or YTGLAA, optionally coupled with a carrier protein.

6. Antibody in accordance with claim 2, wherein bone sialoprotein which is modified in its glycosylation from bone material is used, the donor of which was not capable of normal glycosylation of bone proteins.

7. Antibody in accordance with any one of claims 1 to 6, which are IgY-antibodies from chicken.

8. Antibodies in accordance with any one of claims 1 to 6, which are human or which have been humanised.

9. Process for determining bone sialoprotein from tumour cells in body fluids, particularly in serum, wherein antibodies in accordance with any one of claims 1 to 8 are used.

10. Process in accordance with claim 9 for diagnostic and prognosis of bone metastases.

11. Use of an antibody in accordance with any one of claims 1 to 8 for producing a medicament.

12. Pharmaceutical composition, comprising as an active ingredient antibodies in accordance with any one of claims 1 to 8.

13. Pharmaceutical composition in accordance with claim 12 for prophylaxis and therapy of bone metastases.

14. Use of an antibody in accordance with any one of claims 1 to 8 as means for targeting in producing a diagnostic agent or medicament.

## Revendications

1. Anticorps ou pluralité d'anticorps contre la sialoprotéine osseuse humaine, **caractérisé en ce que** les anticorps lient des épitopes de la sialoprotéine osseuse humaine de cellules de tumeurs dans la région des acides aminés 120 à 135 de la sialoprotéine osseuse humaine (SWISSPROT: SIALJHUMAN, Acc.No. P21815), qui comprennent des acides aminés TGLAA et dont la glycosylation posttranslationale est variée ou incomplète par rapport à de la sialoprotéine osseuse normale des os.

2. Anticorps selon la revendication 1, obtenu avec comme antigène une sialoprotéine osseuse altérée chimiquement ou naturellement dans la glycosylation.

3. Anticorps selon la revendication 1 ou 2, obtenu avec une sialoprotéine osseuse de cellules tumorales comme antigène.

4. Anticorps selon la revendication 3 dans lequel la sialoprotéine osseuse altérée dans la glycosylation est produite dans des cellules tumorales.

5. Anticorps selon la revendication 1, qui est produit contre un antigène peptidique comprenant la séquence d'acides aminés TGLAA ou YTGLAA, éventuellement lié à une protéine-support.

6. Anticorps selon la revendication 2, dans lequel pendant la glycosylation de la sialoprotéine osseuse altérée provenant de tissu osseux est utilisée, dont le donneur n'était pas capable d'une glycosylation normale de protéines osseuses.

7. Anticorps selon une des revendications 1 à 6 qui sont des anticorps IgY de poules.

8. Anticorps selon une des revendications 1 à 6 qui sont humains ou humanisés.

9. Procédé pour déterminer de la sialoprotéine osseuse de cellules tumorales dans des humeurs, en particulier dans du sérum, dans lequel des anticorps selon l'une des revendications 1 à 8 sont utilisés.

10. Procédé selon la revendication 9 pour le diagnostic et le pronostic de métastases osseuses.

11. Utilisation des anticorps selon les revendications 1 à 8 dans la fabrication de médicaments.

12. Composition pharmaceutique comprenant comme substance active des anticorps selon une des revendications 1 à 8.

13. Composition pharmaceutique selon la revendication 12 pour la prophylaxie et la thérapie de métastases osseuses.

14. Utilisation des anticorps selon une des revendications 1 à 8 comme outil de vectorisation pour la production d'un agent diagnostique ou d'un médicament.
